# EUROPEAN PATENT APPLICATION

(11) **EP 3 144 116 A1**
(43) Date of publication of application: **22.03.2017**
(21) Application number: 15792963.9
(22) Date of filing: 15.05.2015
(51) Int. Cl.: B27L 11/00, B09B 3/00, C12P 5/02

(54) **CELLULOSE-BASED BIOMASS JUICING METHOD AND GAS FUEL PREPARATION METHOD**

(30) Priority: 15.05.2014 JP 2014101839
(71) Applicant: IHI Enviro Corporation, Tokyo 135-0042 (JP); Japan International Research Center for Agricultural Sciences, Tsukuba-shi, Ibaraki 305-8686 (JP)
(72) Inventor: YAMASHITA, Masaharu, Tokyo 135-0042 (JP); KOSUGI, Akihiko, Tsukuba-shi Ibaraki 305-8686 (JP)
(74) Representative: Lamb, Martin John Carstairs
(86) International application number: PCT/JP2015/064079
(87) International publication number: WO 2015/174529

(57) **Abstract**

The present invention is a cellulose-based biomass juicing method in which a cellulose-based biomass is chipped, and the cellulose-based biomass is juiced after a predetermined pre-treatment is performed on the cellulose-based biomass, wherein the pre-treatment is a mechanical treatment in which parenchyma constituting the cellulose-based biomass is crushed.

## Description

### [Technical Field]

The present invention relates to a cellulose-based biomass juicing method and a gas fuel preparation method.

Priority is claimed on Japanese Patent Application No. 2014-101839, filed May 15, 2014, the content of which is incorporated herein by reference.

### [Background Art]

As known in the related art, palm oil is a vegetable oil acquired from the fruit of the oil palm. The main production area of such palm oil is Southeast Asia, where it is produced on large-scale farms known as plantations. For example, Patent Document 1 discloses a method (a juicing method) of squeezing sap from a trunk of an oil palm using the trunk as a biomass (resources derived from organisms). Also, Patent Document 1 discloses a shredder for a trunk used in the juicing method and a juicing system including the shredder for the trunk and a squeezing device.

On the other hand, Patent Document 2 also discloses a method (a sap acquisition method) of acquiring sap from the trunk of an oil palm (palm trunks). Such a method of juicing the trunk of an oil palm produces ethanol (bioethanol) or lactic acid through alcohol fermentation or lactic acid fermentation using sugars contained in the sap of the oil palm as a raw material.

In other words, the ratio of the total sap contained in the palm trunk which can be separated through the juicing process is not sufficient, a substantial proportion of sap is discarded together with juice bagasse (pulp) of the palm trunk in the current circumstances, and thus the palm trunk is not sufficiently used as a biomass.

### [Document of Related Art]

### [Patent Document]

[Patent Document 1]
   Japanese Patent No. 4665257
[Patent Document 2]
   Japanese Patent No. 4418871

### [Summary of Invention]

### [Technical Problem]

However, in techniques of Patent Document 1 and Patent Document 2, since a juicing rate of sap is not necessarily sufficient, cost benefit is not found when they are actually applied to plants which produce ethanol or lactic acid. In other words, a ratio of the total sap contained in the palm trunk which can be separated through the juicing process is not sufficient, and a substantial proportion of sap is discarded together with the palm trunk at present. Therefore, a juicing rate of sap needs to be further improved by effectively using palm plants as a biomass in the technical field related to juicing oil palms or palm family plants (palm plants) including such oil palms.

The present invention was made in view of the above-described circumstances, and is for the purpose of improving a juicing rate of sap in various cellulose-based biomasses including cellulose or hemicelluloses, such as palm plants, compared to the related art.

### [Solution to Problem]

In the present invention, in a first aspect related to a cellulose-based biomass juicing method, a cellulose-based biomass is crushed, and the cellulose-based biomass is juiced after a predetermined pre-treatment is performed on the cellulose-based biomass, wherein the pre-treatment is a mechanical treatment in which parenchyma constituting the cellulose-based biomass is crushed.

In a second aspect related to the cellulose-based biomass juicing method of the present invention, in the first aspect, the mechanical treatment is a pulverizing treatment using a mill or a breaking treatment using a cutter and the pulverizing treatment.

In a third aspect related to the cellulose-based biomass juicing method of the present invention, in the second aspect, a water adding process is performed between crushing and the mechanical treatment of the cellulose-based biomass.

In a fourth aspect related to the cellulose-based biomass juicing method of the present invention, in any one of the first to third aspects, the cellulose-based biomass is the trunk of an oil palm.

In a first aspect related to a gas fuel preparation method of a cellulose-based biomass of the present invention, sap acquired through the method of juicing the cellulose-based biomass according to any one of the first to fourth aspects above is subjected to a methane fermentation process.

In a second aspect related to the gas fuel preparation method of a cellulose-based biomass of the present invention, in the first aspect, a saccharification process is performed on a pulp from which the sap is separated through a juicing process, and a saccharified liquid acquired through the saccharification process or the saccharified liquid and the sap are subjected to a methane fermentation process.

### [Effects of Invention]

According to the present invention, since a mechanical treatment in which parenchyma constituting a cellulose-based biomass is crushed is performed as a pre-treatment of a juicing process, a juicing rate of sap included in various cellulose-based biomasses including celluloses or hemicelluloses, such as palm plants, can be improved compared to the related art.

### [Brief Description of Drawings]

Fig. 1 is a flowchart showing processing steps of a cellulose-based biomass juicing method related to an embodiment of the present invention.
Fig. 2A is a schematic diagram of a wet cutter in the embodiment of the present invention.
Fig. 2B is a schematic diagram of a wet mill in the embodiment of the present invention.
Fig. 3 is a schematic diagram showing a tissue form of a palm trunk in the embodiment of the present invention.

### [Description of Embodiments]

Hereinafter, an embodiment of the present invention will be described with reference to the accompanying drawings.

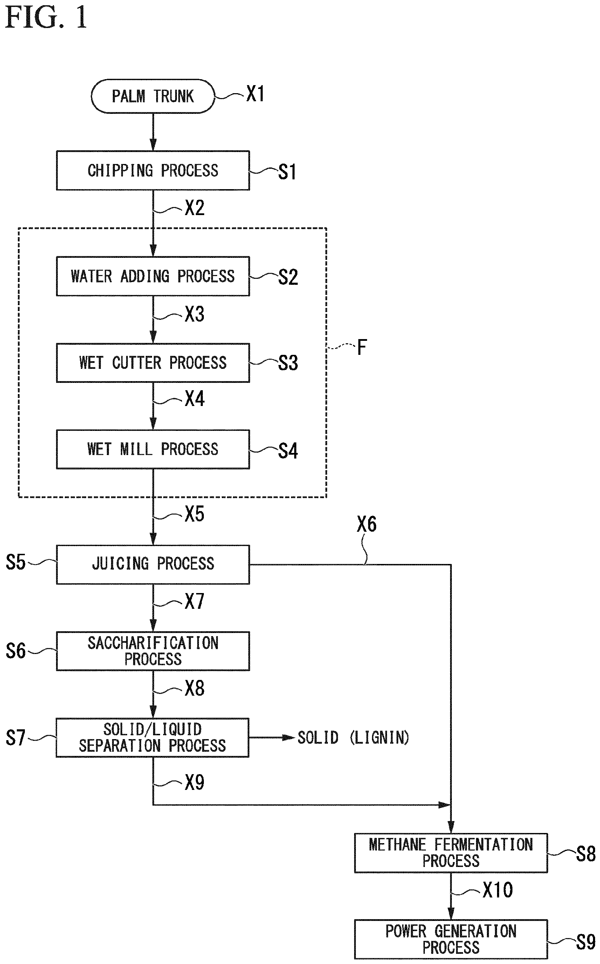

A cellulose-based biomass juicing method related to the embodiment includes selecting a trunk of an oil palm (a palm trunk X1) as a cellulose-based biomass serving as a juicing target and includes steps S1 to S5 in Fig. 1. Also, a gas fuel preparation method of a cellulose-based biomass related to the embodiment includes steps S1 to S8 in Fig. 1.

In a chipping process S1 serving as a first step (a crushing treatment), the log-shaped palm trunk X1 having, for example, a diameter of 30 to 60 cm and a length of about 10 m is crushed into chips (small pieces) having, for example, a maximum dimension of about 2.0 to 3.0 cm. The chipping process S1 is mainly performed in consideration of a wet cutter process S3 in a subsequent stage, that is, the palm trunk X1 is chipped into sizes at which the wet cutter process S3 in the subsequent stage can be adequately performed. Note that a crushing machine used for the chipping process S1 need not be a special device and may be any device as long as it can crush a palm trunk into chips (palm chips X2). The palm trunk X1 may be fragmented into small pieces, shredded, or peeled instead of the palm trunk X1 being chipped.

In a water adding process S2 serving as a second step, water (tap water) which is one to five times a predetermined weight of an aggregate of the palm chips X2 is added to the aggregate at a predetermined weight ratio to produce water-added palm chips X3 are made. Since the palm chips X2 (the palm trunk X1) have an excellent moisture-absorbing property, water is incorporated into solids of the water-added palm chips X3 without being separated into layers of the water and the solids. Moisture is added to the palm chips X2 in the water adding process S2 as well such that the wet cutter process S3 in the subsequent stage can be adequately performed, like in the above-described chipping process S1.

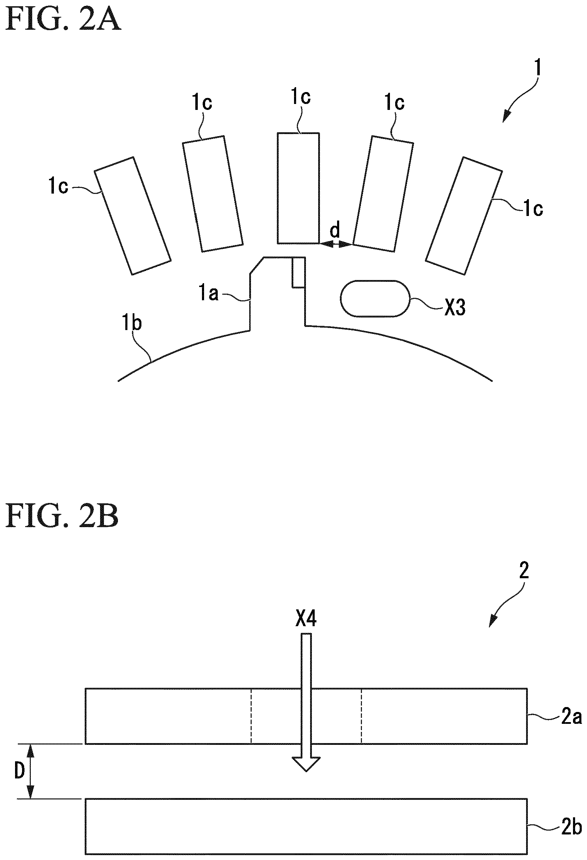

In a wet cutter process S3 serving as a third step, the water-added palm chips X3 are broken to, for example, a maximum dimension of about 0.5 to 1.5 mm. As shown in Fig. 2A, an outer circumference of a wet cutter 1 used in the wet cutter process S3 is provided with a cutting blade 1a, and a plurality of blades 1c are radially provided at the outside of a rotor 1b which rotates in a horizontal plane at an interval (an aperture d). The wet cutter 1 is, for example, a Micro-Meister (Model number: 3M7-40S type) manufactured by MASUKO SANGYO CO., LTD.

The Micro-Meister may have various numbers of blades (the number of blades 1c) and various apertures d, but adaptation of the Micro-Meister in which the number of blades is, for example, 14 and the aperture d is, for example, 1.3 mm is considered. Here, the specification of the wet cutter 1 should be optimized in view of maximizing a juicing rate.

In the wet cutter 1, the water-added palm chips X3 are input onto the rotor 1b or/and into spaces of the rotor 1b with the blades 1c from above, and solids in the water-added palm chips X3 are broken between the cutting blade 1a and a tip (a side end of the rotor 1b) of each of the blades 1c. The solids broken into sizes of the aperture d or less pass through gaps between the blades 1c which are adjacent to each other and are discharged to the outside as broken palms X4.

In a wet mill process S4 serving as a fourth step, the broken palms X4 supplied from the wet cutter 1 together with moisture are ground and pulverized. As shown in Fig. 2B, the wet mill 2 used in the wet mill process S4 includes a rotatable lower grinder 2b which faces an annular upper grinder 2a at a predetermined interval (a clearance D). Also, the clearance D is freely changed in a predetermined range and at a predetermined pitch and is set to, for example, 80 µm. The wet mill 2 is, for example, a Supermasscolloider (model number: MKZB-100J) manufactured by MASUKO SANGYO CO., LTD.

In this wet mill 2, the broken palms X4 are introduced into a site near a center of the annular upper grinder 2a from above, are pulverized between the upper grinder 2a and the lower grinder 2b, and are discharged as pulverized palms X5 in an outer circumferential direction of the lower grinder 2b. In other words, the broken palms X4 acquired in the wet cutter process S3 pass through the wet mill process S4, are fragmented into smaller pieces and become the pulverized palms X5. In other words, a specification of the wet mill 2 should be optimized in view of maximizing the juicing rate.

Also, the wet mill 2 may be a Continuous Vibrating Mill (URAS TECHNO CO., LTD.). The Continuous Vibrating Mill can continuously input and discharge granules without retaining the granules.

Here, the water adding process S2, the wet cutter process S3, and the wet mill process S4 are positioned as a pre-treatment F when the palm chips X2 acquired in the chipping process S1 are processed in a juicing process S5 in the subsequent stage, that is, when sap X6 is separated/harvested from the palm chips X2.

Also, the wet cutter process S3 and the wet mill process S4 in the pre-treatment F are mechanical treatments for physically destroying a water-absorbing structure included in parenchyma by crushing vascular bundles (hard tissue) constituting the palm chips X2 (the palm trunk X1) and the parenchyma which is extremely soft and has a high water-absorbing property compared to the vascular bundles. In the embodiment, the parenchyma is crushed in the wet cutter process S3 and the wet mill process S4 as the pre-treatment of the juicing process S5 so that a juicing rate in the juicing process S5 is improved. Note that the water adding process S2 is positioned as a pre-treatment for the mechanical treatments.

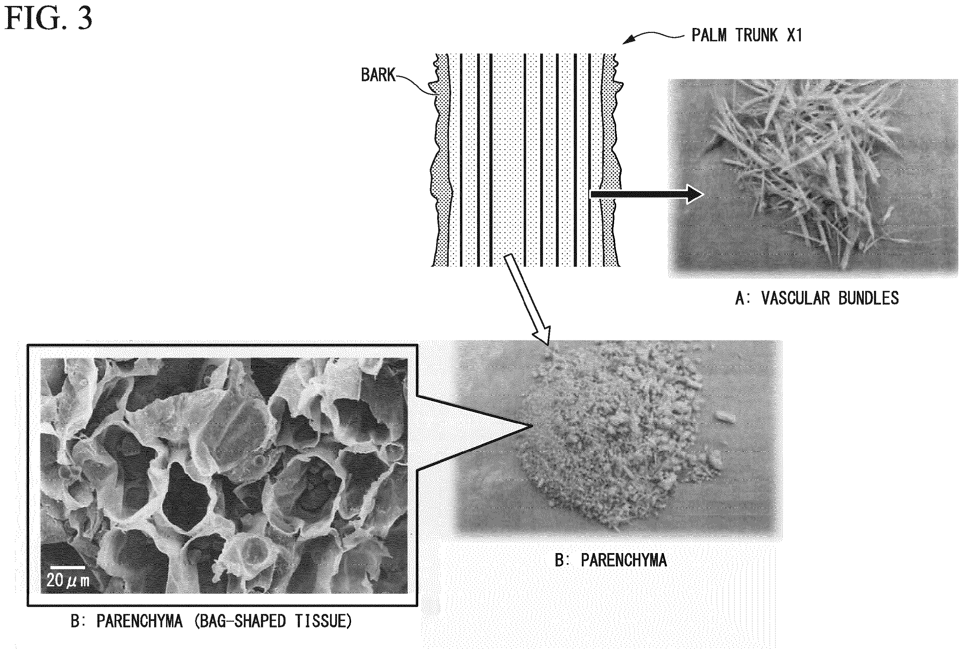

As shown in Fig. 3, the palm trunk X1 includes vascular bundles A and parenchyma B as main portions excluding bark. The vascular bundles A are bundle-shaped tissue passing through the inside of the palm trunk X1 in an axial direction and are responsible for carrying liquids such as water and nutrients and mechanically supporting the palm trunk X1. On the other hand, the parenchyma B is tissue in the vascular bundles A in a direction perpendicular to an axial direction of the palm trunk X1, that is, tissue which adheres the vascular bundles A to each other.

As shown in Fig. 3, the parenchyma B is an aggregate of tissue which is formed in a bag shape (bag-shaped tissue) when viewed under magnification. Sugars (the free sugars pentose and hexose) or starches are accommodated to be wrapped in the bag-shaped tissue. Also, the parenchyma B is tissue having a high water-absorbing property because it has a bag shape. Reviewing a water-absorbing ratio (a weight ratio), the water-absorbing ratio of the parenchyma B is 20.1, the water-absorbing ratio of the vascular bundles is 1.4, the water-absorbing ratio of agar for a medium as a comparison example is 9.8, and the water-absorbing ratio of silica gel is 3.1. The palm trunk X1 includes almost 80% moisture and includes the vascular bundles A and the parenchyma B at a ratio of about 50% as components other than moisture.

In the juicing process S5 serving as a fifth step, the pulverized palms X5 acquired by the wet mill process S4 are separated into solids and liquids. The pulverized palms X5 are a solid/liquid mixture including water originally included in the palm trunk X1 and water added in the water adding process S2, and the solids thereof are fragmented into small pieces up to about 80 µm through the wet cutter process S3 and the wet mill process S4. In the juicing process S5, the pulverized palms X5 are separated into sap X6 and solids (pulp X7) using, for example, a centrifuge or a squeezing device. The sap X6 is a sugar solution which includes water and sugars (the free sugars pentose and hexose) as main components and partially contains starch granules, or the like and is provided as a raw material of a methane fermentation process S8 in the subsequent stage.

The pulverized palms X5 are the solid/liquid mixture which is separated into layers when left to stand for a predetermined time because solids thereof are fragmented into small pieces up to about 80 µm, and are thereby extremely easily separated into solids and liquids. Also, since the parenchyma constituting the palm trunk X1 is sufficiently crushed through the wet cutter process S3 and the wet mill process S4, the moisture-absorbing property is significantly reduced.

In the related art, since a juicing process is performed in a state in which the parenchyma B is maintained without considering the moisture-absorbing property of the parenchyma B, a sufficient juicing rate is not acquired, but in the embodiment, since the juicing process S5 is performed in a state in which the parenchyma B is sufficiently crushed, the juicing rate of the sap X6 can be improved compared to the related art.

Here, with regard to the juicing rate of the sap X6 in the embodiment, separation/harvesting of moisture through the juicing process S5 is not measured through a simple separation rate of moisture from the palm trunk X1, that is, a rate of the total amount of water of the palm trunk X1, but through a rate of the total sugars (the simple sugars pentose and hexose) included in the palm trunk X1. In addition to this, when the total sugars are low, amino nitrogens (free amino acids and proteins) and minerals (calcium, magnesium, etc.) included in the palm trunk X1 may be measured and used as indexes of separation/harvesting.

In the palm trunk X1, sugars are accommodated to be wrapped in the parenchyma B (the bag-shaped tissue), but the sugars in the parenchyma B easily dissolve in moisture when the parenchyma B (the bag-shaped tissue) is crushed through the wet cutter process S3 and the wet mill process S4. Therefore, according to the embodiment, the sugars in the parenchyma B are easily separated from solids as the sap X6 through the juicing process S5.

Since the residual sugars in the solids (the pulp X7) acquired through the juicing process S5 are extremely reduced, the juicing rate is also effectively improved by newly adding water to the pulp X7 and performing solid/liquid separation two times or more.

In a saccharification process S6 serving as a sixth step, low saccharification (single saccharification and/or free saccharification) is performed by hydrolyzing the pulp X7 (juice bagasse) acquired through the juicing process S5. In the saccharification process S6, the simple sugars pentose and hexose are generated by hydrolyzing the pulp X7 on the basis of, for example, an enzyme saccharification method. As known in the related art, cellulose-based biomasses such as the palm trunk X1 or woody biomasses have cellulose, hemicellulose, and lignin as main components. An enzyme saccharification method involves hydrolyzing cellulose and hemicellulose among the main components in the presence of a saccharification enzyme. Also, starch granules included in the parenchyma are converted into solids together in some cases, but hydrolysis can be performed in the presence of amylases or glucoamylases.

Cellulose or hemicellulose does not necessarily need to be decomposed into simple sugars or free sugars in "the saccharification process" in the embodiment, and the pulp X7 (the juice bagasse) may be hydrolyzed to be liquefied or solubilized, and cellulose or hemicellulose may be decomposed into smaller units than simple sugars or free sugars.

In other words, in the saccharification process S6, while the cellulose in the pulp X7 is hydrolyzed to generate hexose, the hemicellulose in the pulp X7 is similarly hydrolyzed to generate pentose. Simple sugars (pentose and hexose) generated through such hydrolysis are soluble in water, and therefore dissolve in water. Therefore, a completely saccharified liquid X8 acquired through the saccharification process S6 is solid/liquid mixed water including solids having lignin as a main component and a saccharified liquid X9 in which simple sugars are dissolved in water. Also, since the vascular bundles A and the parenchyma B constituting the palm trunk are broken and pulverized into small pieces through the pre-treatment F, and surface areas of a cellulose substance and a hemicellulose substance also physically spread, a hydrolysis reaction of enzymes or the like of the cellulose and the hemicellulose in the saccharification process S6 is promoted.

A saccharification process may be a microbial saccharification method using *Clostridium thermocellum.* In particular, the inventors of the present application found that glucan and xylan can be decomposed at 62.5% and 39%, respectively, through a co-culture system of *Clostridium thermocellum* and *Thermoanaerobacter brockii.* For this reason, the saccharification process can be performed through the co-culture system of *Clostridium thermocellum* and *Thermoanaerobacter brockii* with high efficiency.

In a solid/liquid separation process S7 serving as a seventh step, the completely saccharified liquid X8 is separated into solids and liquids. In other words, the saccharified liquid X9 is separated from the completely saccharified liquid X8 using a solid/liquid separation device such as a centrifuge. Also, the saccharified liquid X9 is provided in the methane fermentation process S8 in the subsequent stage as a raw material.

In the methane fermentation process S8 serving as an eighth step, a biogas X10 having methane gas and carbon dioxide as main components is generated through methane fermentation using the sap X6 (the sugar solution) and the saccharified liquid X9 as raw materials. As known in the related art, methane fermentation is a reaction system in which organic matter is decomposed using an anaerobic organic matter decomposition process, that is, action of methane bacteria serving as an anaerobic microorganism, to generate a digestion gas (a biogas) having methane gas and carbon dioxide as main components.

In a power generation process S9 serving as a ninth step, an internal combustion engine is driven using the biogas X10 as a fuel to generate power. In other words, in the power generation process S9, power is generated by burning the biogas X10 using a combustor, and an electric power generator is driven by the power to generate electric power. The electric power generated in the power generation process S9 is used to operate a power source of, for example, the chipping process S1, the water adding process S2, the wet cutter process S3, and the wet mill process S4.

According to the above-described embodiment, since the wet cutter process S3 (the breaking treatment) of the water-added palm chips X3 using the wet cutter 1 and the wet mill process S4 (the pulverizing treatment) of the broken palms X4 using the wet mill 2 are performed as the pre-treatment of the juicing process S5, the juicing rate of the sap X6 in the palm trunk X1 (a palm plant) can be improved compared to the related art.

In the embodiment, the pulp X7 (the juice bagasse) acquired through the juicing process S5 passes through the wet cutter process S3 (the breaking treatment) using the above-described wet cutter and the wet mill process S4 (the pulverizing treatment) using the wet mill and has very smaller sizes than the juice bagasse in the related art. Therefore, according to the embodiment, saccharification efficiency of the pulp X7 in the saccharification process S6 can be significantly improved compared to the related art.

Next, a juicing test will be described. Raw materials (kg) and added water (kg) shown in Table 1 were used as Examples 1 to 9. Apertures (mm) which are indicated were used in a wet cutter. A wet mill had the indicated predetermined interval (clearance D) (µm).

**[Table 1]**

| Example | 1 | 2 | 3 | 4 | 5 | 6 | 7 | 8 | 9 | |
|---|---|---|---|---|---|---|---|---|---|---|
| Raw material (kg) | 10 | 5 | 5 | 2.5 | 2.5 | 2.5 | 2.5 | 2.5 | 5 | |
| Added water (kg) | 0 | 5 | 5 | 5 | 5 | 5 | 5 | 5 | 10 | |
| Wet cutter | S140 | S140 | S140 | S140 | S140 | S140 | S140 | - | - | |
| Aperture (mm) | 1.3 | 1.3 | 1.3 | 1.3 | 1.3 | 1.3 | 1.3 | - | - | |
| Sample No. | 1-A | 2-A | 3-A | 4-A | 5-A | 6-A | 7-A | - | - | |
| Wet mill (µm) | 160 | 160 | 80 | 160 | 80 | 160 | 240 | 240 | 240 | |
| Number of revolutions (rpm) | 1500 | 1500 | 1500 | 1500 | 1500 | 3000 | 3000 | 3000 | 3000 | |
| Sample No. | 1-B | 2-B | 3-B | 4-B | 5-B | 6-B | 7-B | 8-A | 9-A | |
| Wet mill (µm) | - | - | - | | | | | 160 | 80 | 80 |
| Number of revolutions (rpm) | - | - | - | | | | | 3000 | 3000 | 1500 |
| Sample No. | - | - | - | | | | | 8-B | 9-B | 9-C |

Since a wet cutter process and a wet mill process were determined through preliminary tests not to be able to be performed for Examples 1 to 3, the processes were not performed. Also, only tests of a wet mill were performed for Examples 8 and 9.

According to results of Examples 4 to 9, it is understood that an added water ratio needs to be a ratio of a raw material:added water = 1:2. When the added water ratio was the ratio of a raw material:added water = 1:2, a pre-treatment in the wet cutter was not needed, and a process of the wet mill alone was possible.

Moisture in raw chips was discharged from the chips as they were crushed and a reduction in size thereof using the wet cutter and the wet mill progressed, the raw material was converted into slurry, and thus a reduction in viscosity was seen.

Samples which were subjected to the wet cutter and wet mill processes were represented as 4A to 9A, 4B to 9B, and 9C. Raw chips from the samples were squeezed and sap therein was harvested.

A chemical oxygen demand (COD) concentration was 67000 ppm. The water content of the raw chips was 79.86%.

For this reason, an amount of raw chips in 500 g of each sample was 500 g÷3 = 166.7 g. Therefore, calculation of the COD content derived from the raw chips was as follows: 166.7 g×79.86÷100×67000÷1000÷1000 = 8.92 g. Thus, this was the amount of COD which could be theoretically juiced.

Next, weights of supernatants of the samples which were subjected to centrifugation and COD concentrations thereof were measured, and eluted COD concentrations in the supernatants were measured. Note that a COD concentration used in the present application is a COD_{Cr} which is measured using potassium dichromate.

As a result, the COD content of Sample 5B in Example 5 was 75.1%, and the COD content of Sample 9C in Example 9 was 63.6%.

Also, the COD content of Sample 4A in Example 4 was 43.2%, the COD content of Sample 4B in Example 4 was 58.7%, the COD content of Sample 5A in Example 5 was 39.5%, the COD content of Sample 6A in Example 6 was 39.7%, the COD content of Sample 6B in Example 6 was 53.9%, the COD content of Sample 7A in Example 7 was 43.9%, the COD content of Sample 7B in Example 7 was 49.6%, the COD content of Sample 8A in Example 8 was 47.9%, the COD content of Sample 8B in Example 8 was 52.7%, the COD content of Sample 9A in Example 9 was 55.2%, and the COD content of Sample 9B in Example 9 was 52.5%.

According to the above-described results, when a sample was primarily processed using the wet cutter and the wet mill and was secondarily processed using the wet mill to be fragmented into small pieces, COD elution rates into the supernatants were high. This is because a sponge-like structure of parenchyma of an old palm tree shown in Fig. 3 is destroyed so that moisture contained in tissue is reduced.

Note that the present invention is not limited to the above-described embodiment, and, for example, the following modified examples are considered.
(1) The cellulose-based biomass juicing method and the gas fuel preparation method of a cellulose-based biomass related to the above-described embodiment use the palm trunk X1 serving as a type of cellulose-based biomass as the juicing target, but the present invention is not limited thereto. Since cellulose-based biomasses having sap containing sugars include various plants such as crops including palm leaves, bananas, sugar cane, corn, cassava, sago palm, yams, sorghum, potatoes, cellulose and sap (or juice), cellulose/starch/sap (or juice) in addition to the palm trunk X1, the present invention can be applied to various cellulose-based biomasses.
(2) In the cellulose-based biomass juicing method and the gas fuel preparation method of a cellulose-based biomass related to the above-described embodiment, the pre-treatment F including the water adding process S2, the wet cutter process S3, and the wet mill process S4 is performed, but the present invention is not limited thereto. The water adding process S2 may be omitted, and a dry cutter process and a dry mill process may be performed as the pre-treatment F.
(3) In the cellulose-based biomass juicing method and the gas fuel preparation method of a cellulose-based biomass related to the above-described embodiment, the mechanical treatments including the wet cutter process S3 and the wet mill process S4 are performed, but the present invention is not limited thereto. Only the wet mill process S4 may be performed as the mechanical treatment.
(4) In the gas fuel preparation method of a cellulose-based biomass related to the above-described embodiment, the saccharified liquid X9 is acquired from the pulp X7 by performing the saccharification process S6 and the solid/liquid separation process S7, but the present invention is not limited thereto. The saccharification process S6 and the solid/liquid separation process S7 may be omitted so that only the sap X6 is supplied in the methane fermentation process S8.

### [Industrial applicability]

According to the present invention, a juicing rate of sap in various cellulose-based biomasses including cellulose or hemicellulose, such as palm plants, can be improved compared to the related art.

### [Description of Reference Signs]

- 1: Wet cutter
- 1a: Cutting blade
- 1b: Rotor
- 1c: Blade
- 2: Wet mill
- 2a: Upper grinder
- 2b: Lower grinder
- X1: Palm trunk
- X2: Palm chips
- X3: Water-added palm chips
- X4: Broken palms
- X5: Pulverized palms
- X6: Sap
- X7: Pulp
- X8: Completely saccharified liquid
- X9: Saccharified liquid
- X10: Biogas

## Claims

1. A cellulose-based biomass juicing method in which a cellulose-based biomass is crushed, and the cellulose-based biomass is juiced after a predetermined pre-treatment is performed on the cellulose-based biomass, wherein
the pre-treatment is a mechanical treatment in which parenchyma constituting the cellulose-based biomass is crushed.

2. The cellulose-based biomass juicing method according to claim 1, wherein the mechanical treatment is a pulverizing treatment using a mill or a breaking treatment using a cutter and the pulverizing treatment.

3. The cellulose-based biomass juicing method according to claim 2, wherein a water adding process is performed between crushing and the mechanical treatment of the cellulose-based biomass.

4. The cellulose-based biomass juicing method according to any one of claims 1 to 3, wherein the cellulose-based biomass is a trunk of an oil palm.

5. A gas fuel preparation method of a cellulose-based biomass in which sap acquired through the cellulose-based biomass juicing method according to any one of claims 1 to 4 is subjected to a methane fermentation process.

6. The gas fuel preparation method of a cellulose-based biomass according to claim 5, wherein a saccharification process is performed on a pulp from which the sap is separated through a juicing process, and a saccharified liquid acquired through the saccharification process or the saccharified liquid and the sap are subjected to a methane fermentation process.
